(19) **Europäisches Patentamt**

**European Patent Office**

**Office européen des brevets**

(11) **EP 1 481 686 A1**

(12) **EUROPEAN PATENT APPLICATION**

(43) Date of publication:
**01.12.2004 Bulletin 2004/49**

(51) Int Cl.⁷: **A61K 38/19**, A61P 35/00

(21) Application number: **03291247.9**

(22) Date of filing: **26.05.2003**

| | |
|---|---|
| (84) Designated Contracting States:<br>**AT BE BG CH CY CZ DE DK EE ES FI FR GB GR HU IE IT LI LU MC NL PT RO SE SI SK TR**<br>Designated Extension States:<br>**AL LT LV MK** | (72) Inventor: **Rosat, Jean Pierre**<br>**1092 Belmont (CH)** |
| | (74) Representative: **Warcoin, Jacques et al**<br>**Cabinet Régimbeau**<br>**20, rue de Chazelles**<br>**75847 Paris cedex 17 (FR)** |
| (71) Applicant: **Apoxis SA**<br>**1066 Epalinges (CH)** | |

(54) **Use of multimeric ligands of the TNF family with reduced toxicity for treating cell proliferative diseases**

(57)     The present invention concerns the use of a multimerized form of ligands of the TNF family for the preparation of a medicament for injection into an appropriate cavity of the body, for the treatment of diseases wherein cell proliferation has to be controlled wherein the ligand of the TNF family is selected among Fas ligand, CD40L, TRAIL and APRIL.

EP 1 481 686 A1

**Description**

The present invention relates to a new method for the administration of ligands of the TNF family with reduced toxicity.

Members of the TNF receptor family and their cognate ligands have been recognized to play a major role in the control of the balance between cell proliferation and cell death in mammals. Most functions associated with the ligand/receptor system of the members of the TNF family are in relation with the control of cell proliferation, differentiation and apoptosis. Imbalance between cell death and cell proliferation can lead to various pathological conditions such as autoimmune diseases, inflammatory diseases and cancer.

Receptors of the TNF family and their ligands (cytokines) have been widely studied in the past decades and are well known in the art (Bodmer & al., TIBS, Vol. 27, No. 1, January 2002, pp. 19-27; Locksley & al., Cell 104, 487-501 (2001); Gruss and Dower, Blood, 85:3378-3404 (1995); see bibliographic parts in US application No. 20020123116, paragraphs 2-10 and US application No. 20020006391).

The receptors of the TNF receptor family are type I transmembrane proteins. They all share a typical structure of cell surface receptors with an N-terminal extracellular domain, a transmembrane and an intracellular domain. Homology identified between family members has been found mainly in the extracellular domain ("ECD") comprising repetitive cysteine-rich patterns. TNF receptor family proteins are also usually cleaved proteolytically to release soluble receptor ECDs that can function as inhibitors of the cognate cytokines (Nophar, Y. et al., EMBO J., 9:3269 (1990); and Kohno, T. et al., Proc. Natl. Acad. Sci. U.S.A., 87:8331 (1990)).

In contrast to their receptors, cytokines of the TNF family are type II transmembrane proteins, whose C-terminus is an extracellular globular head. Some cytokines of the TNF family are cleaved proteolytically at the cell surface to form a homotrimeric molecule that functions as a soluble cytokine.

Receptors of the TNF family form homotrimers when bound to their ligand (Cha & al., J. Biol. Chem. 275, 31171-31177 (2000); Hymowitz & al., Moll. Cell 4, 563-571 (1999); Mongkolsapaya & al., Nat. Struct. Biol. 6, 1048-1053 (1999)).

Several receptors of the TNF family and their cognate ligands have been identified and disclosed with a variety of different nomenclatures. The TNF Receptor Superfamily has been recently organized where the symbols for the receptor genes are based upon their relationship with the ligands:

- http://www.gene.ucl.ac.uk/nomenclature/genefamily/tnfrec2.html. Ligands are well known in the art and disclosed in various publications:
- http://www-personal.umich.edu/~ino/List/996.htm;
- http://www.gene.ucl.ac.uk/nomenclature/genefamily/tnflig.html comprising the following ligands:

| LTA | lyphotoxin alpha (TNF superfamily, member 1) | TNFSF1, TNFB, LT |
|---|---|---|
| TNF | tumor necrosis factor (TNF superfamily, member 2) | TNFSF2, TNFA, DIF |
| LTB | lyphotoxin beta (TNF superfamily, member 3) | TNFSF3, TNFC, p33 |
| TNFSF4 | tumor necrosis factor (ligand) superfamily, member 4 (tax-transcriptionally activated glycoprotein 1, 34kD) | OX-40L, gp34, TXGP1 OX-40L, gp34, TXGP1 |
| TNFSF5 | tumor necrosis factor (ligand) superfamily, member 5 (hyper-IgM syndrome) | CD40LG, IMD3, HIGM1, CD40L, hCD40L, TRAP, CD154, gp39 |
| TNFSF6 | tumor necrosis factor (ligand) superfamily, member 6 | FasL, APT1LG1 |
| TNFSF7 | tumor necrosis factor (ligand) superfamily, member 7 | CD70, CD27L, CD27LG |
| TNFSF8 | tumor necrosis factor (ligand) superfamily, member 8 | CD30LG |
| TNFSF9 | tumor necrosis factor (ligand) superfamily, member 9 | 4-1BB-L |
| TNFSF10 | tumor necrosis factor (ligand) superfamily, member 10 | TRAIL, Apo-2L, TL2 |

(continued)

| TNFSF11 | tumor necrosis factor (ligand) superfamily, member 11 | TRANCE, RANKL, OPGL, ODF |
|---|---|---|
| TNFSF12 | tumor necrosis factor (ligand) superfamily, member 12 | TWEAK, DR3LG, APO3L |
| TNFSF13 | tumor necrosis factor (ligand) superfamily, member 13 | APRIL |
| TNFSF14 | tumor necrosis factor (ligand) superfamily, member 14 | LIGHT, LTg, HVEM-L |
| TNFSF15 | tumor necrosis factor (ligand) superfamily, member 15 | TL1, VEGI |
| TNFSF18 | tumor necrosis factor (ligand) superfamily, member 18 | AITRL TL6 hGITRL |

Products and methods of treatment of diseases associated with disorders in the TNF family ligand/receptor interaction have been disclosed in the art, comprising administration of antibodies or ligands for the treatment of rheumatoid arthritis or Chron's disease.

Multimeric forms of such ligands have also been disclosed in the art, and more specifically multimeric forms comprising at least six soluble fractions of the ligand bounds to a multimerization tail (WO 01/49866). Such multimeric forms, also called Megaligands are agonists of the membrane-bounds cytonkines and are inducing cell death. These Megaligands have been disclosed for the treatment of various diseases where cell proliferation has to be controlled, including Neoplasia, benign and malignant tumors (including malignant mesothelioma, metastatic ovary carcinoma, glioblastoma, metastasic colon cancer), autoimmune diseases and autoinflammatory diseases.

It was found however that due to their efficacy, some of these molecules may present high risks of toxicity depending upon their administration route. For instance, injection i.v. of a Mega-FasL (hexamer of the Fas ligand extracellular soluble fraction) to a mice, may result in almost immediate death of the mice through hepato-toxicity and liver failure.

It has been found now that injection of multimerized forms of ligands of the TNF family to specific "geographical" areas of the body could substantially reduce the toxicity of the same multimerized ligands, allowing the treatment of pathologies in these "geographical" areas of the body wherein cell proliferation has to be controlled.

The "geographical" areas of the body according to the invention are cavities of the body where organs are separated from the remaining of the body with a barrier or membrane. Once injected into the cavity, the membrane or barrier of the cavity and its cellular components will prevent the multimerized form of the ligand to substantially migrate into the general blood stream where it could affect essential organs such as the liver. Such cavities include the peritoneal cavity, the pleural cavity, the pericardial cavity, the respiratory tract (upper and lower airways), the upper digestive tract (including the mouth), the urinary tract (including bladder), the articular space and the central nervous system.

The present invention therefore concerns a new method of treatment of diseases wherein cell proliferation has to be controlled comprising the injection of multimerized forms of ligands of the TNF family into appropriate cavities of the body, the ligand being selected amongst Fas-ligand, CD40L, TRAIL and APRIL.

More particularly, the present invention relates to the use of a multimerized form of ligands of the TNF family for the preparation of a medicament for injection into an appropriate cavity of the body, for the treatment of diseases wherein cell proliferation has to be controlled wherein the ligand of the TNF family is selected among Fas ligand, CD40L, TRAIL and APRIL.

Appropriate cavities are cavities of the body where the disease-associated cell proliferation has to be controlled.

Diseases or pathologies of the above cavities include all pathologies comprising cell proliferation. It includes more particularly pathologies where cell death has to be induced for its control and/or treatment, such as tumors in the above cavities, primary tumors, like glioblastomas or mesothelioma (pleural and peritoneal) or secondary tumors from any cancer forms giving metastasis in the above cavities, such as ovarian metastatic cancers and colo-rectal cancers.

The multimerized forms of ligands of the TNF family comprise at least four, globular soluble extracellular fractions of the ligands of the TNF family, preferably at least five, more preferably at least six, even more preferably six globular soluble extracellular fractions of the ligands of the TNF family bounds to a multimerization moiety.

In a preferred embodiment of the invention, the multimerized form of ligand of the TNF family is an hexamer comprising six monomers, assembled together, each of the monomers comprising a polypeptide of formula (I):

$$H-L \quad (I)$$

wherein

L represents a C-terminal ligand moiety, comprising the soluble extracellular fraction of a ligand of the TNF family selected among Fas ligand, CD40L, TRAIL and APRIL, and

H represents a N-terminal hexamerization moiety.

According to the present invention, the ligand moiety L includes the "full length" of the soluble extracellular fraction of a ligand and biologically functional fragments of the same fraction. "Biologically functional fragments" are fragments of a soluble extracellular fraction of a ligand of the TNF family conserving their ability to bind to the same receptor(s), with substantially the same affinity.

L preferably comprises the full length extracellular soluble fraction of the above ligands.

According to an embodiment of the invention, L comprises the extracellular domain of human FAS ligand (hFasL), comprising amino acids Glu 139 to Leu 281 of hFasL.

Hexamers according to the invention are either "true" hexamers, dimers of trimers or trimers of dimers. In the first case, H is a hexamerization polypeptide HP. In the latter cases, H comprises two moieties, a first moiety consisting of a dimerization polypeptide (DP) and a second moiety consisting of a trimerization polypeptide (TP).

The polypeptides according to the present invention comprise a polypeptide represented by one the following formulas (Ia), (Ib) and (Ic):

$$HP - L \quad (Ia) \ (\text{"true" hexamers}),$$

$$DP\text{-}TP - L \quad (Ib) \ (\text{trimers of dimers}), \text{ and}$$

$$TP\text{-}DP - L \quad (Ic) \ (\text{dimers of trimers})$$

wherein L, HP, DP and TP are defined above and below.

Examples of HP, TP and DP are well known in the art and comprise isolated peptide fragments of natural hexameric, trimeric or dimeric polypeptides, the said isolated fragments being responsible for the hexamerization, dimerization or trimerization of the said natural hexamers, dimers or trimers.

Such molecules are well known in the art and comprises polypeptides of the collectin family, such as the ACRP30 or ACRP30-like proteins (WO96/39429, WO 99/10492, WO 99/59618, WO 99/59619, WO 99/64629, WO 00/26363, WO 00/48625, WO 00/63376, WO 00/63377, WO 00/73446, WO 00/73448 or WO 01/32868), apM1 (Maeda et al., Biochem. Biophys. Res. Comm. 221: 286-9, 1996), C1q (Sellar et al., Biochem. J. 274: 481-90, 1991), or C1q like proteins (WO 01/02565), which proteins comprise "collagen domains" consisting in collagen repeats Gly-Xaa-Xaa'.

Other oligomerized polypeptides are known in the art, including polypeptides with a "coiled-coil" domains (Kammerer RA, Matrix Biol 1997 Mar;15(8-9):555-65; discussion 567-8; Lombardi & al., Biopolymers 1996;40(5): 495-504; http://mdl.ipc.pku.edu.cn/scop/data/scop.1.008.001.html), like the Carilage Matrix Protein (CMP) (Beck & al., 1996, J. Mol. Biol., 256, 909-923), , or polypeptides with a dimerization domain, like polypeptides with a leucine zipper or osteoprotegerin (Yamaguchi & al., 1998).

According to a specific embodiment of the invention, HP comprises the hexamerization domains of A, B or C chains of polypeptides of the C1q family.

TP are known in the art and comprise the trimerization domains (C-terminal moiety) of CMP (i.e. GeneBank 115555, amino acids 451-493) or the trimerization domain of ACRP30 and ACRP30-like molecules. According to a preferred embodiment of the present invention, TP comprises a stretch of collagen repeats.

According to the invention, a "stretch of collagen repeats" consists in a series of adjacent collagen repeats of formula (II):

-   $(\text{Gly-Xaa-Xaa'})_n$- (II)

wherein Xaa and Xaa' represents independently an amino acid residue, and n represents an integer from 10 to 40.

Xaa and Xaa' are preferably selected independently among natural amino acids such as Ala, Arg, Asn, Asp, Cys, Gln, Glu, Gly, His, Ile, Leu, Lys, Met, Phe, Pro, Ser, Thr, Trp, Tyr or Val.

Xaa preferably represents independently an amino acid residue selected among Ala, Arg, Asp, Glu, Gly, His, Ile, Leu, Met, Pro or Thr, more preferably Arg, Asp, Glu, Gly, His or Thr.

Xaa' preferably represents independently an amino acid residue selected among Ala, Asn, Asp, Glu, Leu, Lys, Phe, Pro, Thr or Val, more preferably Asp, Lys, Pro or Thr.

When Xaa' represents a Pro residue, the collagen repeat Gly-Xaa-Pro is designated to be a "perfect" collagen repeat, the other collagen repeats being designated as "imperfect".

According to a preferred embodiment of the invention, the stretch of collagen repeats comprises at least 1 perfect collagen repeat, more preferably at least 5 perfect collagen repeats.

According to a preferred embodiment of the invention, n is an integer from 15 to 35, more preferably from 20 to 30, most preferably 21, 22, 23 or 24.

According to the present invention, the stretch of collagen repeat may comprise up to three "non collagen residues" inserted between two adjacent collagen repeats. These "non collagen residues" consist in 1, 2 or 3 amino acid residues, provided that when the "non collagen residue" consists in 3 amino acids residues, the first amino acid is not Gly.

According to a preferred embodiment of the invention, TP consists in an uninterrupted stretch of 22 collagen repeats. More preferably, TP consists in the stretch of 22 collagen repeats of the sequence below, corresponding to amino acids 45 to 110 of mACRP30, as represented in SEQ ID NO 2 of WO 96/39429: **Gly** Ile **Pro Gly** His **Pro Gly** His Asn **Gly** Thr **Pro Gly** Arg Asp **Gly** Arrg Asp **Gly** Thr **Pro Gly** Glu Lys **Gly** Glu Lys **Gly** Asp Ala **Gly** Leu Leu **Gly** Pro **LLys Gly** Glu Thr **Gly** Asp Val, **Gly** Met Thr **Gly** Ala Glu **Gly** Pro Arg **Gly** Phe **Pro Gly** Thr **Pro Gly** Ar Lys **Gly** Glu **Pro Gly** Glu Ala

According to another preferred embodiment of the invention, TP consists in the stretch of 22 collagen repeats corresponding to amino acids 42 to 117 of hACRP30, as represented in SEQ ID NO 7 of WO 96/39429.

DP are known in the art and comprises dimerization fragments of immunoglobulins (Fc fragments), the C-terminal dimerization domain of osteoprotegerin (Receptor: δN-OPG; amino acids 187-401), or polypeptides sequences comprising at least 6, preferably 8 to 30 amino acids and allowing dimerization. These peptides generally comprise at least a cysteine residue allowing the formation of disulfide bonds. Other polypeptides useful as DP according to the invention are peptides designated as "leucine zippers" comprising a Leucine residue being present every seventh residue.

Examples of such peptides comprising at least one cysteine residue comprise the following peptides:

Val Asp Leu Glu Gly Ser Thr Ser Asn Gly Arg Gln Cys Ala Gly Ile Arg Leu

Glu Asp Asp Val Thr Thr Thr Glu Glu Leu Ala Pro Ala Leu Val Pro Pro Pro Lys Gly Thr Cys Ala Gly Trp Met Ala

Gly His Asp Gln Glu Thr Thr Thr Gln Gly Pro Gly Val Leu Leu Pro Leu Pro Lys Gly Ala Cys Thr Gly Trp Met Ala.

The second sequence above corresponds to amino acids 17 to 44 of mACRP30 as represented in SEQ ID NO 2 of WO 96/39429, and the third sequence above corresponds to amino acids 15 to 41 of SEQ ID NO 7 of WO 96/39429.

Other peptides comprising at least one cysteine residue, can be found in amino acid sequences upstream the stretch of collagen repeats of molecules having a structure analogous to ACRP30 (ACRP30-like) as disclosed in WO 99/10492, WO 99/59618, WO 99/59619, WO 99/64629, WO 00/26363, WO 00/48625, WO 00/63376, WO 00/63377, WO 00/73446, WO 00/73448 or WO 01/32868.

Leucine zippers are well known in the art and can be found in natural proteins and eventually identified using bioinformatics tools available to the one skilled in the art (http://www.bioinf.man.ac.uk/zip/faq.shtml; http://2zip.molgen. mpg.de/; Hirst, J.D., Vieth, M., Skolnick, J. & Brooks, C.L. III, *Predicting Leucine Zipper Structures from Sequence,* Protein Engineering, 9, 657-662 (1996)).

The constitutive elements L, H, HP, TP and/or DP in the polypeptides of formula I, Ia, Ib or Ic, according to the invention, are assembled by peptides bonds. They may be separated by "linkers" which will not affect the functionality of the polypeptide according to the invention, its ability to form hexamers and to bind with the receptor corresponding to the ligand L. Such linkers are well known in the art of molecular biology.

The polypeptide according to the invention may also comprise peptide sequences on its N-terminus and/or C-terminus, which will not affect the functionality of the polypeptide according to the invention. These peptides may comprise affinity tags, for purification or detection of the polypeptide according to the invention. Such affinity tags are well known in the art and comprise a FLAG peptide (Hopp et al., Biotechnology 6: 1204 (1988)) or a Myc-His tag.

According to a preferred embodiment of the invention, H comprises a dimerization polypeptide (DP) and a trimerization polypeptide (TP), and is most preferably represented by the following formula:

DP-TP - L (Ib)

wherein R, DP and TP are defined above and below.

More preferably, DP and TP represent together amino acids 17 to 110 of mACRP30 as represented in SEQ ID NO 2 of WO 96/39429 or amino acids 15 to 107 of hACRP30 as represented in SEQ ID NO 7 of WO 96/39429.

In preferred embodiment of the invention the polypeptide comprises the fusion polypeptide selected among mACRP30:hFasL, mACRP30:hTRAIL, and mACRP30:hCD40L. Such polypeptides and their preparation are disclosed in WO 01/49866 which content is incorporated herein by reference.

According to another embodiment of the invention, the hexamerization moiety comprises a Fc portion of IgG comprising amino acids 248 to 473 of gi2765420, as disclosed in the PCT application No. PCT/EP02/09354, which content is incorporated herein by reference.

In the method according to the invention, the multimerized forms of ligands are injected in the form of an pharmaceutical composition comprising the said multimerized forms of ligands in a pharmaceutically acceptable carrier suitable for its administration by injection.

Suitable carriers, adjuvant, preservatives, etc., used prepare pharmaceutical compositions, are well-known to those in the art (Gennaro (ed.), Remington's Pharmaceutical Sciences, 19th Edition (Mack Publishing Company 1995)).

The multimerized forms of ligands according to the invention are administered to the patient in a manner such that their concentration is sufficient to bind their cognate receptors and induce cell death.

As a preferred embodiment of the present invention, the pharmaceutical composition comprises from 0.1 to 100 weight % of multimerized forms of ligands according to the invention, based on the total weight of the pharmaceutical composition, more preferably from 2.5 to 100 %. When the composition according to the invention comprises 100 % multimerized forms of ligands, it is preferably in a lyophilized form.

The multimerized form of ligands is administered from 1 to 4 times daily, at a level sufficient to achieve a total daily dose of 0.0001 to 0.2 mg/Kg/day, preferably 0.001 to 0.1 mg/kg/day.

In the method according to the invention, the multimerized forms of ligands can be used alone or in combination with one or more other mean of treatment.

By "mean of treatment" it is understood according to the invention to comprise other molecules or compositions suitable for the treatment of the same diseases, but also other means of treatment known in the art of treatment of the same diseases such as radiation therapy, chemotherapy, or eventually surgery.

Other molecules or compositions suitable for the treatment of the same diseases are well known in the art, such as any of the molecules or compositions listed under the heading "Cancerologie" in the Dictionaire Vidal (2003 ed.), in the Merk Index or in the Physician Desk Reference.

Other molecules or compositions also comprise such molecules or composition able to enhance the cell sensibility to apoptosis. Intracellular proteins involved in the control of cell death are known in the art, such as the FLIP molecules disclosed in WO 98/44104. It was shown that overexpression of FLIP in cells could inhibit apoptosis. A contrario, inhibition of FLIP or inhibition of FLIP expression could enhance the cell sensibility to apoptosis. Molecules inhibiting FLIP or FLIP expression are known in the art such as antisens molecules, disclosed in WO 98/44104 or interfering RNA molecules (RNAi or dsRNA) prepared according to methods well known in the art, such as methods disclosed in WO 00/44895, WO 02/55692 or WO 02/55693, which content is incorporated herein by reference. In the method according to the invention, the multimerized forms of ligands can be injected in combination with such an antisens or dsRNA inhibiting FLIP expression.

An injection or "use" in combination with another mean according to the invention comprises the use simultaneously, separately or sequencally of the multimerized form of the ligand and the other mean. For a simultaneous use, the multimerized form of the ligand and the other mean, preferably another molecule or composition, can be injected together in the same pharmaceutical composition, or in two separated compositions mixed together in an extemporaneous manner before injection. For a sequential administration, the multimerized form of the ligand and the other mean, preferably another molecule or composition, are in two distinct compositions. The other molecule or composition may be used according to conventional methods of administration, such as orally or by injection iv.

**Examples**

The invention is further described in the following examples.

Except as otherwise described, all examples are carried out using standard techniques, which are well known to a person skilled in the art of molecular and/or cellular biology (i.e. T. Maniatis, E. F. Fritsch. J. Sambrook, Molecular cloning, 1982. ;M. Ausubel et al., Current Protocols in Molecular Biology, Eds., Wiley, New York, 2000).

**Testing Mega-FasL toxicity in mice injected ip versus iv Study design:**

Female Balb/c mice 8-10 weeks old will be assigned to one of the 10 treatment groups as indicated below. Mice will receive ip or iv either saline solution or Mega-FasL. Mice will be injected on Day 1 and will be bled after 2h, 6h, 24h and 48h or ALT and AST quantification. After 48h, survival will be measured and mice will be sacrificed.

| Group | Number | Route | Treatment |
|---|---|---|---|
| 1 | 6 | iv | PBS 200µl |
| 2 | 6 | iv | Mega-FasL (100µg/kg) 2µg in 200µl |
| 3 | 6 | iv | Mega-FasL (50µg/kg) 1µg in 200µl |
| 4 | 6 | iv | Mega-FasL (25µg/kg) 0.5µg in 200µl |
| 5 | 6 | iv | Mega-FasL (12.5µg/kg) 0.25µg in 200µl |
| 6 | 6 | ip | PBS 200µl |
| 7 | 6 | ip | Mega-FasL (100µg/kg) 2µg in 200µl |
| 8 | 6 | ip | Mega-FasL (50µg/kg) 1µg in 200µl |
| 9 | 6 | ip | Mega-FasL (25µg/kg) 0.5µg in 200µl |
| 10 | 6 | ip | Mega-FasL (12.5µg/kg) 0.25µg in 200µl |

**Liver function tests:**

Bleeding mice 2h, 6h, 24h and 48 hours post injection. (Day of injection is Day 0) Collect blood (200µl) into an eppendorf tube containing 20µl of heparin (Liquémine Roche). Centrifuge the eppendorf 3 min 5000 rpm in a desktop centrifuge Collect the plasma into a new tube, and store it at -80°C Results represented on the Tables below. Table 1 represents the ALT levels in mice at given times (2, 6, 24 and 48h) post injection of Mega-FasL iv (groups 1 to 5). Table 2 represents the ALT levels in mice at given times (2, 6, 24 and 48h) post injection of Mega-FasL ip (groups 6 to 10).

Table 1

| Group | ALT U/l | | | |
|---|---|---|---|---|
| | 2h | 6h | 24h | 48h |
| 1 | 119+/- 46 | 120+/-41 | 100+/-72 | 57+/-16 |
| 2 | 1281+/-464 | 3000 | | |
| 3 | 1085+/-267 | 5544+/-2615 | 5433+/-2366 | 1323+/-887 |
| 4 | 97+/-37 | 1450+/-1047 | 413+/-211 | 95+/-25 |
| 5 | 166+/-195 | 196+/-127 | 112+/-81 | 43+/-5 |

Table 2

| Group | ALT U/l | | | |
|---|---|---|---|---|
| | 2h | 6h | 24h | 48h |
| 6 | 75+/-29 | 168+/-39 | 95+/-32 | 55+/-21 |
| 7 | 97+/-21 | 268+/-130 | 409+/-227 | 96+/-44 |
| 8 | 136+/-89 | 301+/-178 | 245+/-152 | 42+/-10 |
| 9 | 96+/-55 | 183+/-100 | 194+/-81 | 56+/-8 |
| 10 | 137+/-75 | 173+/-110 | 143+/-66 | 44+/-5 |

The ALT levels at given time are more than 10 times lower after injection i.p. according to the invention compared to injection i.v. Reference ALT levels are between 35 and 51 U/l. Survival at 48h was measured and reported in the Table 3 below.

Table 3

| Group | Number | Route | Treatment | Survival % |
|---|---|---|---|---|
| 1 | 6 | iv | PBS 200µl | 100 |
| 6 | 6 | ip | PBS 200µl | 100 |
| **2** | **6** | **iv** | **Mega-FasL (100µg/kg)** | **0** |
| **7** | **6** | **ip** | **Mega-FasL (100µg/kg)** | **100** |
| **3** | **6** | **iv** | **Mega-FasL (50µg/kg)** | **30** |
| **8** | **6** | **ip** | **Mega-FasL (50µg/kg)l** | **100** |
| 4 | 6 | iv | Mega-FasL (25µg/kg) | 100 |
| 9 | 6 | ip | Mega-FasL (25µg/kg) | 100 |
| 5 | 6 | iv | Mega-FasL (12.5µg/kg)l | 100 |
| 10 | 6 | ip | Mega-FasL (12.5µg/kg) | 100 |

ALT levels in the serum represent the liver function. Increased ALT levels are specifically indicating liver damage. Therefore, the results presented on Table 2 indicate that Mega-FasL injected ip according to the present invention causes a mild liver dysfunction, while Mega-FasL injected iv causes severe liver dysfunction that can lead to the death of the animal, as seen in groups 2 and 3.

Administration of multimerized forms of ligands of the TNF family according to the invention allows the administration of higher doses of ligands, with low levels of ALT and 100% survival when 0% is observed at the same dose but injected iv.

**Claims**

1. Use of a multimerized form of ligands of the TNF family for the preparation of a medicament for injection into an appropriate cavity of the body, for the treatment of diseases wherein cell proliferation has to be controlled wherein the ligand of the TNF family is selected among Fas ligand, CD40L, TRAIL and APRIL.

2. The use as claimed in claim 1, wherein the cavity is selected among the peritoneal cavity, the pleural cavity, thepericardial cavity , the respiratory tract (upper and lower airways), the upper digestive tract (including the mouth), the urinary tract (including bladder), the articular space and the central nervous system.

3. The use as claimed in one of claims 1 or 2, wherein the diseases or pathologies of the above cavities includes pathologies where cell death has to be induced for its control and/or treatment.

4. The use as claimed in claim 3, wherein the diseases or pathologies comprises tumors in the said cavities, primary tumors, like glioblastomas or mesothelomia (pleural and peritoneal) or secondary tumors from any cancer forms giving metastasis in the cavities, such as ovarian metastatic cancers and colo-rectal cancers.

5. The use as claimed in one of claims 1 to 4, wherein the multimerized form of ligands of the TNF family comprises at least four, soluble extracellular fractions of the ligands of the TNF family, preferably at least five, more preferably at least six, even more preferably six soluble extracellular fractions of the ligands of the TNF family bounds to a multimerization moiety.

6. The use as claimed in one of claims 1 to 5, wherein the multimerized form of ligand of the TNF family is an hexamer comprising six monomers, assembled together, each of the monomers comprising a polypeptide of formula (I):

H-L (I)

wherein L represents a C-terminal ligand moiety, comprising the soluble extracellular fraction of a ligand of the TNF family, and H represents an N-terminal hexamerization moiety.

7. The use as claimed in one of claims 1 to 6, wherein L is Fas ligand.

8. The use as claimed in claim 7, wherein L comprises the extracellular domain of human Fas ligand (hFasL), comprising amino acids Glu 139 to leu 281 of hFasL.

**European Patent Office**

**EUROPEAN SEARCH REPORT**

Application Number

EP 03 29 1247

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,A | WO 01 49866 A (APOTECH RES & DEV LTD ;HOLLER NILS (CH); TSCHOPP JUERG (CH); SCHNE) 12 July 2001 (2001-07-12) * page 19, line 15 - page 23, line 3 * | 1-8 | A61K38/19 A61P35/00 |
| A | HOLLER NILS ET AL: "Two adjacent trimeric Fas ligands are required for Fas signaling and formation of a death-inducing signaling complex." MOLECULAR AND CELLULAR BIOLOGY, vol. 23, no. 4, February 2003 (2003-02), pages 1428-1440, XP002258597 ISSN: 0270-7306 (ISSN print) * abstract * | 1-8 | |
| A | SCHNEIDER P ET AL: "APOPTOSIS INDUCED BY DEATH RECEPTORS" PHARMACEUTICA ACTA HELVETIAE, XX, XX, vol. 74, no. 2/3, March 2000 (2000-03), pages 281-286, XP001121938 ISSN: 0031-6865 * the whole document * | 1-8 | |
| D,A | BODMER J-L ET AL: "The molecular architecture of the TNF superfamily" TIBS TRENDS IN BIOCHEMICAL SCIENCES, ELSEVIER PUBLICATION, CAMBRIDGE, EN, vol. 27, no. 1, 1 January 2002 (2002-01-01), pages 19-26, XP004332356 ISSN: 0968-0004 * the whole document * | | TECHNICAL FIELDS SEARCHED (Int.Cl.7) A61K |

The present search report has been drawn up for all claims

| Place of search | Date of completion of the search | Examiner |
|---|---|---|
| MUNICH | 21 October 2003 | Deck, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 03 29 1247

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2003

| Patent document cited in search report | Publication date | Patent family member(s) | Publication date |
|---|---|---|---|
| WO 0149866 A | 12-07-2001 | DE 19963859 A1 | 12-07-2001 |
| | | AU 2367301 A | 16-07-2001 |
| | | BR 0017054 A | 07-01-2003 |
| | | CA 2395632 A1 | 12-07-2001 |
| | | WO 0149866 A1 | 12-07-2001 |
| | | EP 1246925 A1 | 09-10-2002 |
| | | HU 0203628 A2 | 28-02-2003 |
| | | JP 2003518949 T | 17-06-2003 |
| | | US 2003053984 A1 | 20-03-2003 |

For more details about this annex : see Official Journal of the European Patent Office, No. 12/82